# EUROPEAN PATENT APPLICATION

(11) **EP 4 670 758 A1**
(43) Date of publication of application: **31.12.2025**
(21) Application number: 24184713.6
(22) Date of filing: 26.06.2024
(51) Int. Cl.: A61M 5/24, A61C 19/08

(54) **ONE-STEP ANESTHETIC BUFFERING CARPULE**

(71) Applicant: Sadek, Yasser E., Palmdale, CA 93550 (US)
(72) Inventor: Sadek, Yasser E., Palmdale, CA 93550 (US)
(74) Representative: Dilg, Haeusler, Schindelmann Patentanwaltsgesellschaft mbH

(57) **Abstract**

A one-step anesthetic buffering carpule is advantageously disposed within a syringe so as to provide a pH-raising buffer that mixes with the carpule anesthetic just prior to injection. A puncture unit is disposed within a rubber plunger containing the buffer. The puncture unit is manually actuated to detach a rubber plunger seal so as to mix the buffer with the anesthetic.

## Description

This application relates to a one-step anesthetic buffering carpule.

### BACKGROUND OF THE INVENTION

Medical personnel, including dentists and physicians, often inject an anesthetic into a patient prior to performing various procedures. However, numbing solutions, such as lidocaine, marcain, carbocain and septicain sting when injected, due to their low (acidic) pH. In order to reduce the sting, a buffering solution, such as sodium bicarbonate, can be mixed into the anesthetic prior to injection so as to increase the pH. This reduces injection pain and allows the anesthetic to take effect quicker.

### SUMMARY OF THE INVENTION

**A** one-step anesthetic buffering carpule provides the most convenient and fastest method for a doctor to deliver a buffered anesthetic to a patient than any available product on the market today. A carpule delivery system is familiar to all dentists and can be used with reusable syringes that are in current use. A carpule contains enough sodium bicarbonate solution to buffer an anesthetic contained in the carpule. The buffer is released when the chamber is activated by pressing a puncture unit This breaks a thin bladder located on the bottom of a rubber plunger, so as to combine the anesthetic and buffer compounds. A needle is mounted to the syringe, and the rubber plunger is pushed into the carpule so as to extrude the buffered anesthetic into a patient. A soft rubber plug fills the top of the puncture unit, allowing the syringe harpoon to fixedly engage the puncture unit so that the dentist may aspirate the syringe.

The carpule delivery system comprises a plastic carpule, a pliable, ribbed-rubber chamber and a plastic puncture unit. The carpule has a sealed first-end and open second-end. The sealed end has a small-circular protrusion for attaching a disposable injection needle. The open second-end receives the ribbed-rubber chamber, which is inserted fully into the carpule. The chamber contains enough sodium bicarbonate solution to buffer the anesthetic. The plastic puncture unit has a first-end comprising a soft rubber plug that fills the top of the puncture unit. This allows the syringe harpoon to fixedly engage the puncture unit so that the dentist may aspirate the syringe. The plastic puncture unit has an opposite second end comprising a pair of harpoons.

The buffer is released when the buffer chamber is collapsed or otherwise activated by pressing the puncture unit The puncture unit harpoons breach the thin bladder located on the bottom of a rubber plunger so as to combine the anesthetic and buffer compounds. A needle is mounted to the syringe, and the rubber plunger is pushed into the carpule so as to extrude the buffered anesthetic into a patient.

The plunger is injection molded with a softer rubber forming the bladder and a harder rubber forming the outer ribbed portion of the plunger. The plunger ribs help seal the carpule as well as allow the plunger to easily slide down the carpule interior. The plastic carpule is a standard carpule used in dentistry except that the tip is sealed with soft rubber that allows a dental needle to be inserted in it after the puncture unit breaches the plunger so as to buffer the anesthetic.

### BRIEF DESCRIPTION OF THE DRAWINGS

**FIG. 1** illustrates of a one-step anesthetic buffering carpule;
**FIGS. 2A-C** are side perspective, bottom perspective and side cross-sectional illustrations, respectively, of a puncture unit;
**FIG. 3** is a side perspective view of a rubber plunger;
**FIG. 4** is an exploded view of the puncture unit, the plunger and the carpule; and
**FIGS. 5A-B** illustrate a one-step buffering carpule before and after activation.

### DETAILED DESCRIPTION OF THE PREFERRED EMBODIMENTS

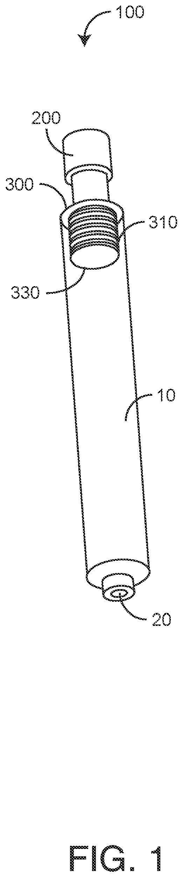

**FIG. 1** generally illustrates an advantageous one-step anesthetic buffering carpule 100 having a plastic carpule 10, a puncture unit 200 and a rubber plunger 300. A buffer (not visible) is disposed within the rubber plunger 300. The plunger 300 is injection molded with two different strength rubbers. A softer rubber forms the bladder 330 and a harder rubber forms the outer ribbed portion 310 of the plunger 300. The ribbed portion 310 helps seal the carpule 10 as well as allow the plunger 300 to slide down the carpule 10. The carpule 10 is the standard size used in dentistry except that the carpule has a soft rubber 2 seal 20 that allows a dental needle (not shown) to be inserted in it after the puncture unit and the plunger unit are activated.

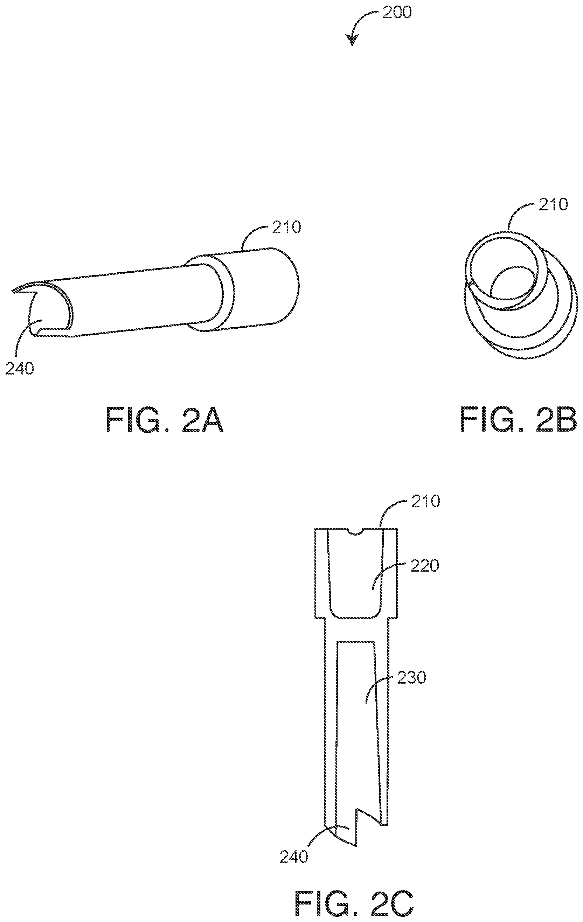

**FIGS. 2A-C** illustrates the puncture unit 200, which has a knob 210 disposed at a first end and sharp points 240 disposed at an opposite second end. A soft rubber plug 220 is added to the interior of the puncture unit knob 210 to allow for the dentist's syringe harpoon (not shown) to engage the knob 210. This allows the dentist to aspirate the syringe if need be.

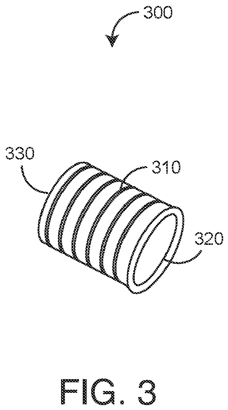

**FIG. 3** illustrates a rubber plunger 300 having a ribbed portion 310, an open portion 320 that receives the puncture unit 200 (FIGS. 2A-C) and a soft rubber bladder 330. As noted above, a buffer is disposed within the rubber plunger 300. In addition, the rubber plunger seals the anesthetic disposed within the carpule 10. In a particular embodiment, the plunger 300 is filled with 0.1 ml of a 7% solution of sodium bicarbonate and the carpule is filed with 1.5 ml of a 2% solution of lidocaine.

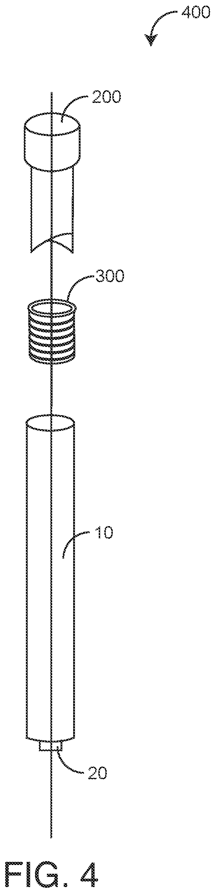

**FIG. 4** further illustrates the puncture unit 200, the rubber plunger 300, the carpule 10 and the soft rubber seal 20.

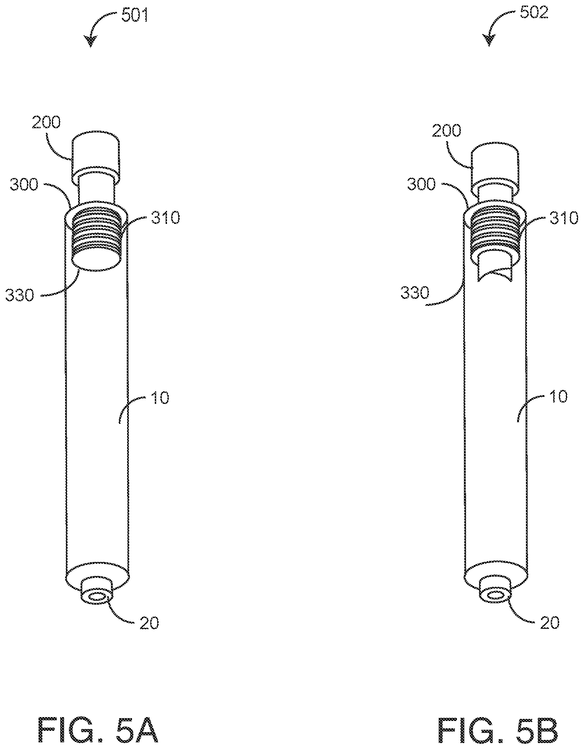

**FIGS. 5A-B** illustrate the one-step anesthetic buffering carpule 100 before activation of the one-step anesthetic buffering carpule 100 (FIG. 5A) and after activation.

A one-step anesthetic buffering carpule is has been disclosed in detail in connection with various embodiments. These embodiments are disclosed by way of examples only and are not to limit the scope of the claims that follow. One of ordinary skill in the art will appreciate many variations and modifications.

## Claims

1. A one-step anesthetic buffering carpule comprising:
a carpule having a first end and a second end;
a liquid anesthetic contained within the carpule;
a capsule at least partially disposed within the carpule proximate the first end so as to fluidly seal the first end;
a buffer contained within the capsule: and
an activator at least partially disposed proximate the buffer and extending from the carpule first end.

2. The one-step anesthetic buffering carpule according to claim 1 wherein the capsule is a rubber plunger fully disposed within the carpule first end.

3. The one-step anesthetic buffering carpule according to claim 2 further comprising:
a rubber seal disposed at a carpule second end for receiving a needle; and
the activator is disposed within the rubber plunger and extends from the carpule first end.

4. The one-step anesthetic buffering carpule according to claim 3 wherein the activator is a puncture unit actuated from a first position to a second position so as to release the anesthetic buffer from the rubber plunger and combine the anesthetic buffer with the anesthetic.

5. The one-step anesthetic buffering carpule according to claim 4 wherein the puncture unit is manually actuated to release the anesthetic buffer into an anesthetic.

6. The one-step anesthetic buffering carpule according to claim 5 wherein the puncture unit has a generally larger cylindrical first end extending from the carpule and a generally smaller cylindrical second end disposed within the rubber plunger.

7. The one-step anesthetic buffering carpule according to claim 6 wherein the generally smaller end of the puncture unit is configured to tear the rubber plunger bottom so as to release the buffer into the anesthetic.

8. The one-step anesthetic buffering carpule according to claim 7 further comprising a pair of sharp harpoons disposed on smaller end of the puncture unit so as to tear the rubber plunger bottom when manually actuated.

9. The one-step anesthetic buffering carpule according to claim 8 further wherein the puncture unit is actuated with thumb pressure on the generally larger cylindrical first end.

10. The one-step anesthetic buffering carpule according to claim 9 wherein the buffered anesthetic is manually shaken after the puncture unit is actuated.

11. A one-step anesthetic buffering method comprising:
disposing a rubber plunger within a carpule first end;
containing a liquid buffer within the rubber plunger; and
sealing the liquid buffer with a first end of a puncture unit.

12. The one-step anesthetic buffering method according to claim 11 further comprising disposing a plurality of harpoon points on the first end of the plunger unit.

13. The one-step anesthetic buffering method according to claim 12 further comprising actuating the puncture unit from a first position to a second position so as to unseal the second end of rubber plunger and buffer with the anesthetic.

14. The one-step anesthetic buffering apparatus according to claim 13 further comprising:
a rubber seal disposed at a carpule second end for receiving a needle; and
a puncture unit is disposed within the rubber plunger and extends from the carpule first end.

15. The one-step anesthetic buffering apparatus according to claim 14 wherein:
an anesthetic buffer is disposed within the rubber plunger; and
the puncture unit is manually actuated to release the anesthetic buffer into an anesthetic.

## Amended claims

### Amended claims in accordance with Rule 137(2) EPC.

1. A one-step anesthetic buffering carpule (100) comprising:
a carpule (10) having a first end and a second end;
a liquid anesthetic contained within the carpule (10);
a capsule (300) at least partially disposed within the carpule (10) proximate the first end so as to fluidly seal the first end;
a buffer contained within the capsule (300);
an activator (200) at least partially disposed proximate the buffer and extending from the carpule first end; and
comprising a pair of sharp harpoons (240) disposed on smaller end of the puncture unit (200) so as to tear the rubber plunger bottom when manually actuated.

2. The one-step anesthetic buffering carpule (100) according to claim 1 wherein the capsule is a rubber plunger (300) fully disposed within the carpule first end.

3. The one-step anesthetic buffering carpule (100) according to claim 2 further comprising:
a rubber seal (20) disposed at a carpule second end for receiving a needle; and
the activator (200) is disposed within the rubber plunger (300) and extends from the carpule first end.

4. The one-step anesthetic buffering carpule (100) according to claim 3 wherein the activator is a puncture unit (200) actuated from a first position to a second position so as to release the anesthetic buffer from the rubber plunger (300) and combine the anesthetic buffer with the anesthetic.

5. The one-step anesthetic buffering carpule (100) according to claim 4 wherein therubber plunger has a bladder portion and an outer ribbed portion, wherein
the bladder portion is softer than the outer ribbed portion .

6. The one-step anesthetic buffering carpule (100) according to claim 5 wherein the puncture unit (200) has a generally larger cylindrical first end extending from the carpule (10) and a generally smaller cylindrical second end disposed within the rubber plunger (300).

7. The one-step anesthetic buffering carpule (100) according to claim 6 wherein the generally smaller end of the puncture unit (200) is configured to tear the rubber plunger bottom so as to release the buffer into the anesthetic.

8. The one-step anesthetic buffering carpule (100) according to claim 7 wherein the two harpoons are positioned around a perimeter of the smaller cylindrical second end of the puncture unit.

9. The one-step anesthetic buffering carpule (100) according to claim 8 further wherein the puncture unit (200) is actuated with thumb pressure on the generally larger cylindrical first end.

10. The one-step anesthetic buffering carpule (100) according to claim 9 wherein the buffered anesthetic is manually shaken after the puncture unit (200) is actuated.

11. A one-step anesthetic buffering method comprising:
disposing a rubber plunger (300) having an open first end and a closed second end within a carpule containing an anesthetic;
containing a liquid buffer within the rubber plunger (300); and
sealing the liquid buffer in the rubber plunger (300) with a puncture unit (200),
wherein the puncture unit (200) has a first end and a second end, the second end sized to sealingly fit within an open first end of the rubber plunger (300);
actuating the puncture unit (200) from a first position to a second position so as to unseal the closed second end of rubber plunger (300) by depressing the puncture unit (200) through the rubber plunger (300) so that two harpoons positioned at a portion of the second end of the puncture unit (200) pierce the closed second end of the rubber plunger (300) thereby mixing the liquid buffer with the anesthetic.

12. The one-step anesthetic buffering method according to claim 11 further comprising:
a rubber seal (20) disposed at a carpule second end for receiving a needle for extracting the buffered anesthetic from the carpule (10) .
